# EUROPEAN PATENT SPECIFICATION

(11) **EP 4 351 513 B1**
(45) Date of publication and mention of the grant of the patent: **15.04.2026**
(21) Application number: 22731157.8
(22) Date of filing: 30.05.2022
(51) Int. Cl.: A61K 8/67, A61K 8/73, A61Q 19/00

(54) **SKIN CARE COMPOSITION**
HAUTPFLEGEZUSAMMENSETZUNG
COMPOSITION POUR LE SOIN DE LA PEAU

(30) Priority: 11.06.2021 WO PCT/CN2021/099576; 03.08.2021 EP 21189246
(43) Date of publication of application: 17.04.2024
(73) Proprietor: Unilever IP Holdings B.V., 6708 WH Wageningen (NL); Unilever Global IP Limited, Wirral, Merseyside CH62 4ZD (GB)
(72) Inventor: BIAN, Wei, 6708 WH Wageningen (NL); WEI, Ping, 6708 WH Wageningen (NL)
(74) Representative: Unilever Patent Group
(86) International application number: PCT/EP2022/064522
(87) International publication number: WO 2022/258408

(56) References cited:
- EP-A1- 3 818 973
- WO-A1-2017/194486
- US-A1- 2018 161 259
- DATABASE GNPD [online] MINTEL; 8 April 2011 (2011-04-08), ANONYMOUS: "Anti-Wrinkle Gel Cream", XP055880560, retrieved from https://www.gnpd.com/sinatra/recordpage/1527416/ Database accession no. 1527416
- DATABASE GNPD [online] MINTEL; 8 April 2011 (2011-04-08), ANONYMOUS: "Anti-Wrinkle Gel Cream", XP055880560, retrieved from https://www.gnpd.com/sinatra/recordpage/1527416/ Database accession no. 1527416

## Description

### Field of the invention

The present invention relates to a skin care composition comprising retinoid. In particular, the present invention is related to a skin care composition comprising retinoid, a first modified starch and a second modified starch, wherein the weight ratio of the total modified starches to the retinoid is at least 7:1.

### Background of the invention

Retinoids (e.g. retinol and retinyl esters) are common ingredients used in skin care products. Retinol (vitamin A) is an endogenous compound which occurs naturally in the human body and is essential for normal epithelial cell differentiation. Natural and synthetic vitamin A derivatives have been used extensively in the treatment of a variety of skin disorders and have been used as skin repair or renewal agents. Retinoic acid has been employed to treat a variety of skin conditions, e.g., acne, wrinkles, psoriasis, age spots and discoloration.

DATABASE GNPD [Online], MINTEL; 8 April 2011 (2011-04-08), anonymous: "Anti- Wrinkle Gel Cream", XP055880560, Database accession no. 1527416 is said to disclose an anti-wrinkle gel comprising retinyl palmitate, retinyl acetate, hydroxypropyl starch phosphate, and aluminium starch octenylsuccinate. EP 3 818 973 A1 (Thorel, Jean-Noël, NAOS Institute of Life Science) is said to disclose a composition comprising hydroxypropyl starch phosphate and sodium starch octenylsuccinate. WO 2017/194486 A1 (Unilever) discloses a composition comprising a retinoic acid precursor and a skin benefit agent comprising a resorcinol and/or a derivative thereof. US 2018/161259 A1 (The Procter & Gamble Company) is said to disclose personal care compositions in the form of an oil-in-water emulsion containing a stable retinoid.

However, sometimes there are some difficulties when incorporating retinoids into skin care compositions, for example the stability of the product at higher temperature may be affected by the inclusion of retinoids, perhaps due to the interaction of retinoids with the ingredients in the skin care composition.

We have recognised that there remains a need to provide a skin care composition containing retinoids which is even stable after storage at high temperature. It was surprisingly found that by incorporating a combination of modified starches into the skin care composition with specific ratio, it is able to achieve a stable composition after storage at high temperature.

### Summary of the invention

In a first aspect, the present invention is directed to a skin care composition comprising retinoid, a first modified starch and a second modified starch, wherein the weight ratio of the total modified starches to the retinoid is at least 7:1, the retinoid comprises retinol, retinyl ester, or a mixture thereof, the first modified starch is nonionic starch, and the second modified starch is anionic starch.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

The composition of the present invention comprises a retinoid. The retinoid comprises retinol, retinyl ester, or a mixture thereof and preferably the retinoid is selected from retinol, retinyl ester, or a mixture thereof.

The term "retinol" includes the following isomers of retinol: all-trans-retinol, 13-cis-retinol, 11-cis-retinol, 9-cis-retinol, 3,4-didehydro-retinol, 3,4-didehydro-13-cis-retinol; 3,4-didehydro-11-cis-retinol; 3,4-didehydro-9-cis-retinol. Preferred isomers are selected from all-trans-retinol, 13-cis-retinol, 3,4-didehydro-retinol, and 9-cis-retinol. Most preferred retinol is all-trans-retinol, due to its wide commercial availability.

Retinyl ester is an ester of retinol. The term "retinol" has been defined above. Retinyl esters suitable for use in the present invention are preferably C₁-C₃₀ esters of retinol, more preferably C₂-C₂₀ esters of retinol, and most preferably C₂, C₃, and C₁₆ esters of retinol. Examples of retinyl esters comprises retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecanoate, retinyl laurate, retinyl tridecanoate, retinyl myristate, retinyl pentadecanoate, retinyl heptadecanoate, retinyl stearate, retinyl isostearate, retinyl nonadecanoate, retinyl arachidonate, retinyl behenate, retinyl linoleate, retinyl oleate or a combination thereof. The retinyl ester for use in the present invention is preferably selected from retinyl palmitate, retinyl acetate, retinyl linoleate, retinyl oleate, retinyl propionate or a mixture thereof. More preferably the retinyl ester is selected from retinyl palmitate, retinyl acetate, retinyl propionate, or a mixture thereof. Most preferably the retinyl ester is selected from retinyl palmitate, retinyl propionate, or a mixture thereof.

Particularly preferred retinoid is selected from all-trans-retinol, retinyl palmitate, retinyl acetate, retinyl propionate, or a mixture thereof. Most preferably the retinoid is selected from retinyl palmitate, retinyl propionate, or a mixture thereof.

Preferably, retinoid is employed in the composition in an amount of 0.0001 to 5% by weight of the composition, more preferably in an amount of 0.005 to 3%, even more preferably from 0.05 to 1% and most preferably in an amount of 0.1 to 0.5% by weight of the composition.

Preferably, the total amount of retinyl palmitate and retinyl propionate in the composition is from 0.0001 to 5% by weight of the composition, more preferably from 0.005 to 3%, even more preferably from 0.05 to 1% and most preferably from 0.1 to 0.5% by weight of the composition.

The composition of the present invention comprises a first and second modified starches. For sake of clarity, the first modified starch is different from the second modified starch. Modified starch refers to starch having a structure altered from the native state of the raw starch. Raw starch preferably comprises corn, potato, rice, waxy maize, wheat, sago and/or tapioca starches which has not been chemically modified. The modified starch may be modified by enzyme, oxidation, cross-linking reaction, and/or substitution from raw starch. Preferably, the first and second modified starches are chemically modified starches.

The first modified starch is nonionic starch. Nonionic starches mean modified starches that has been modified in a way such that they carry a neutral charge. Preferably, the nonionic starch is obtainable by esterification and/or etherification of raw starch. Preferably, the first modified starch comprises oxidized starch, acetylated starch, hydroxyethylated starch, hydroxypropylated starch, phosphorylated starch, starch phosphates, starch sulfates, allyl starch, benzyl starch, carbamoylethyl starch, carboxymethyl starch, cyanomethyl starch, or a mixture thereof. More preferably the first modified starch comprises hydroxyethylated starch, hydroxypropylated starch, acetylated starch, hydroxyethylated starch, hydroxypropylated starch, starch phosphate, starch sulfate or a combination thereof. Even more preferably the first modified starch comprises hydroxypropylated starch, hydroxyethylated starch, starch phosphate, starch sulfate or a combination thereof. Most preferably, the first modified starch is hydroxypropyl starch phosphate.

Preferably, the first modified starch is present in amount of 0.01 to 8%, more preferably 0.05 to 4% and most preferably 0.2 to 2% by weight of the composition. Preferably, the hydroxypropyl starch phosphate is present in amount of 0.01 to 8%, more preferably 0.05 to 4% and most preferably 0.2 to 2% by weight of the composition.

The second modified starch is anionic starch. Anionic starches mean modified starches that have been modified in a way such that they carry a negative charge. Preferably, the second modified starch comprises starch modified by succinate and/or substituted succinate. More preferably, the second modified starch comprises aluminium starch octenylsuccinate, sodium starch octenylsuccinate, calcium starch octenylsuccinate, sodium carboxymethyl starches, sodium starch glycolate or a mixture thereof. Even more preferably the second modified starch comprises aluminium starch octenylsuccinate, sodium starch octenylsuccinate or a combination thereof. Most preferably, the second modified starch is aluminium starch octenylsuccinate.

Preferably, the second modified starch is present in amount of 0.01 to 12%, more preferably 0.2 to 8% and most preferably 0.5 to 5% by weight of the composition. Preferably, the aluminium starch octenylsuccinate is present in amount of 0.01 to 12%, more preferably 0.2 to 8% and most preferably 0.5 to 5% by weight of the composition.

Preferably, the weight ratio of the first modified starch to the second modified starch is 1:30 to 5:1, more preferably 1:15 to 2:1 and most preferably 1:10 to 1:1. Preferably, the weight ratio of the hydroxypropyl starch phosphate to the aluminium starch octenylsuccinate is 1:30 to 5:1, more preferably 1:15 to 2:1 and most preferably 1:10 to 1:1.

Preferably, the weight ratio of the total modified starches to the retinoid is 7:1 to 200:1, more preferably 7:1 to 100:1, and most preferably 8:1 to 50:1. Preferably, the weight ratio of the total amount of hydroxypropyl starch phosphate and aluminium starch octenylsuccinate to the retinoid is 7:1 to 200:1, more preferably 7:1 to 100:1, and most preferably 8:1 to 50:1. Preferably, the weight ratio of the total amount of hydroxypropyl starch phosphate and aluminium starch octenylsuccinate to the total amount of retinyl palmitate and retinyl propionate is 7:1 to 200:1, more preferably 7:1 to 100:1, and most preferably 8:1 to 50:1.

The composition preferably comprises a fatty ester. Fatty ester as used herein refers to an ester having a straight chain with length of at least 6 carbon atoms, preferably at least 8 carbon atoms. In addition, the fatty ester can be a straight chain fatty ester, a branched chain fatty ester, a benzoate ester, or a combination thereof. Preferably, the fatty ester is liquid at 25 °C and atmospheric pressure.

Preferably, the fatty ester comprises fatty monoester, fatty polyester or a mixture thereof. Preferably, the fatty ester comprises a combination of fatty monoester and fatty polyester. Fatty monoester refers to monoester having a straight chain with length of at least 6 carbon atoms, preferably at least 8 carbon atoms. Fatty polyester refers to **polyester** having a straight chain with length of at least 6 carbon atoms, preferably at least 8 carbon atoms. Preferably, the fatty monoester is monoester of C₈₋₂₄ fatty acid with C₁₋₈ monovalent alcohol or monoester of C₁₋₆ organic acid with C₆₋₂₀ monovalent alcohol. More preferably, the fatty monoester is monoester of C₁₀₋₂₀ fatty acid with C₁₋₆ monovalent alcohol or monoester of C₂₋₄ organic acid with C₈₋₁₈ monovalent alcohol. Preferably, the fatty polyester is ester of C₆₋₂₀ fatty acid with C₂₋₈ polyol or C₂₋₈ diol. More preferably the fatty polyester is ester of C₈₋₁₈ fatty acid with C₂₋₆ polyol or C₂₋₆ diol.

Preferably, the fatty monoester is selected from myristyl propionate, cetyl acetate, cetyl propionate, isodecyl neopentanoate, isopropyl myristate, isopropyl palmitate, isopropyl laurate, methyl laurate, methyl linoleate, methyl myristate, methyl stearate, methyl palmitate, isopropyl isostearate, butyl stearate, isobutyl palmitate, butyl myristate, ethyl palmitate, ethyl myristate, isobutyl stearate, isobutyl myristate, or a mixture thereof. More preferably, the fatty monoester comprises myristyl propionate, cetyl propionate, isodecyl neopentanoate, isopropyl myristate, isopropyl palmitate, methyl myristate, methyl stearate, or a mixture thereof. Even more preferably, the fatty monoester comprises isopropyl myristate. Most preferably the fatty monoester is isopropyl myristate. Preferably, the amount of fatty monoester is present in amount of 2 to 32%, more preferably 5 to 25%, even more preferably 8 to 18% by weight of the composition.

Preferably fatty polyester comprises capric/caprylic triglyceride, sunflower seed oil, cotton oil, canola oil, soybean oil, castor oil, borage oil, olive oil, shea butter, jojoba oil and mixtures thereof. More preferably the fatty polyester comprises capric/caprylic triglyceride, and most preferably the fatty polyester is capric/caprylic triglyceride. Preferably, the amount of fatty polyester is present in amount of 0.2 to 22%, more preferably 1 to 15%, even more preferably 3 to 10% by weight of the composition.

To improve the stability and/or reduce the discoloration of the composition, it is preferable that the weight ratio of the fatty monoester to the retinoid is at least 12:1. More preferably the weight ratio of the fatty monoester to the retinoid is 12:1 to 300:1, even more preferably, 15:1 to 150:1 and most preferably 20:1 to 70:1. Preferably, the weight ratio of the fatty ester to the retinoid is at least 18:1. More preferably the weight ratio of the fatty ester to the retinoid is 18:1 to 500:1, even more preferably, 20:1 to 350:1 and most preferably 25:1 to 100:1. Preferably, the weight ratio of the total fatty esters to the total modified starches is no greater than 9:1, more preferably 1:2 to 9:1, even more preferably 1:2 to 8:1, and most preferably 1:1 to 8:1.

Preferably, the total amount of fatty ester is present in amount of 3 to 45%, more preferably 5 to 36%, even more preferably 12 to 28% by weight of the composition.

The composition may comprise resorcinol derivative. Resorcinol derivative preferably refers to that at least one hydrogen on the ring structure and/or on a hydroxy group of the resorcinol replaced with an alkyl group, phenyl alkyl group. Preferably, the resorcinol derivative is 4-substituted resorcinol. Preferably, the resorcinol derivative is selected from 4-ethyl resorcinol, 4-butyl resorcinol, 4-hexyl resorcinol, phenylethyl resorcinol, or a mixture thereof, and more preferably, the resorcinol derivative comprises 4-hexyl resorcinol. The amount of the resorcinol derivative is preferably in the range of 0.00001 to 10%, more preferably from 0.001 to 5% and most preferably from 0.1 to 0.6% by weight of the total amount of the composition.

Preferably, the composition comprises Vitamin B3 compounds (including derivatives of vitamin B3). The vitamin B3 compounds comprises niacin, nicotinic acid, niacinamide or a mixture thereof. The most preferred vitamin B3 compound is niacinamide. Amount of Vitamin B3 compounds may be 0.1 to 10%, preferably 0.5 to 5% by weight of the composition.

Preferably, the composition comprises polyhydric alcohol. Polyhydric alcohols may be selected from group of glycerin, propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. Most preferred polyhydric alcohol is glycerol known also as glycerin. The amount of polyhydric alcohol may range anywhere from 0.1 to 20%, preferably 0.5 to 15% and more preferably 2 and 10% by weight of the composition.

The composition may comprise water in amount of 30 to 98% by weight of the composition, more preferably from 35 to 95%, even more preferably from 42 to 88%, most preferably from 50 to 82% by weight of the composition.

Preferably, the composition has a viscosity of at least 10 cSt, more preferably in the range 100 to 500,000 mPa·s, even more preferably 300 to 100,000 mPa·s, and most preferably 1,000 to 20,000 cSt, when measured by Brookfield viscometer (T-B spindle, 5 rpm, 30 seconds) at 25°C. Preferably, the composition is in the form of fluid.

Preferably, the composition is an emulsion, more preferably an oil-in-water emulsion. The skin care composition refers to a composition suitable for topical application to human skin, including leave-on and wash-off products but preferably leave-on compositions. The term "leave-on" as used with reference to compositions herein means a composition that is applied to or rubbed on the skin, and left thereon. The term "wash-off" as used with reference to compositions herein means a skin cleanser that is applied to or rubbed on the skin and rinsed off substantially immediately subsequent to application. Preferably the skin care composition means a fluid liquid, and particularly a moisturizer rather than a make-up product. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

The following examples are provided to facilitate an understanding of the invention. The examples are not intended to limit the scope of the claims.

### Examples

### Example 1

This example demonstrates the effect of the presence of the combination of two modified starch.

**Table 1**

| Ingredient | Samples (wt%)* | | | | |
|---|---|---|---|---|---|
| | A | B | C | D | 1 |
| Water | To 100 | To 100 | To 100 | To 100 | To 100 |
| Retinyl Propionate | 0.32 | 0.32 | 0.32 | 0.32 | 0.32 |
| PEG-100 Stearate | 3.00 | - | - | - | - |
| Montanov^{™} 68 MB ¹ | - | 3.00 | - | - | - |
| StarDesign^{™} Care ² | - | - | 3.00 | - | - |
| C*EmTex ^{™} 06328 ³ | - | - | - | 3.00 | - |
| StarDesign^{™} Power ⁴ | - | - | - | - | 3.00 |
| Xanthan Gum | 0.20 | 0.20 | 0.20 | 0.20 | 0.20 |
| Isopropyl Myristate | 10.00 | 10.00 | 10.00 | 10.00 | 10.00 |
| Hexylresorcinol | 0.40 | 0.40 | 0.40 | 0.40 | 0.40 |
| Niacinamide | 3.00 | 3.00 | 3.00 | 3.00 | 3.00 |
| Capric/Caprylic Triglyceride | 5.00 | 5.00 | 5.00 | 5.00 | 5.00 |
| Glycerin | 2.00 | 2.00 | 2.00 | 2.00 | 2.00 |
| 1,3-Butylene glycol | 3.00 | 3.00 | 3.00 | 3.00 | 3.00 |
| Cetyl Alcohol | 0.50 | 0.50 | 0.50 | 0.50 | 0.50 |
| Disodium EDTA | 0.05 | 0.05 | 0.05 | 0.05 | 0.05 |
| Antioxidant | 0.20 | 0.20 | 0.20 | 0.20 | 0.20 |
| Preservative | 0.65 | 0.65 | 0.65 | 0.65 | 0.65 |

| | | | | | |
|---|---|---|---|---|---|
| *The level refers the level of the listed ingredient. 1: Montanov^{™} 68 MB has INCI name of: Cetearyl Alcohol (and) Cetearyl Glucoside, supplied by Air Liquide. 2: StarDesign^{™} Care has INCI name of Hydroxypropyl starch phosphate, supplied by Cargill. 3: C*EmTex ^{™} 06328 is Starch sodium octenyl succinate supplied by Cargill. 4. StarDesign^{™} Power, contains 10-40% of hydroxypropyl starch phosphate and 60-90% of sodium starch octenylsuccinate by weight, supplied by Cargill. | | | | | |

A series of skin care compositions were formulated according to Table 1 by following standard procedures.

The performances of the samples were evaluated by the procedure as follows: all samples were put into same transparent bottles with identical amount. These transparent bottles with samples inside were placed into oven at 55°C for 24 hours. Then, these transparent bottle with samples inside were taken out, observed, and recorded in Table 2.

**Table 2.**

| Sample | Before storage at 55°C | After storage at 55°C |
|---|---|---|
| A | Stable emulsion | Phase separation |
| B | Stable emulsion | Phase separation |
| C | Stable emulsion | Phase separation |
| D | Stable emulsion | Phase separation |
| 1 | Stable emulsion | Stable emulsion |

It was surprisingly found that only by incorporating a combination of modified starches into a skin care composition containing retinyl propionate, a stable emulsion is capable of being achieved even after storage at high temperature.

### Example 2

This example demonstrates the effect of the weight ratio of the total amount of modified starches to retinoid.

**Table 3**

| Ingredient | Samples (wt%)* | | | | |
|---|---|---|---|---|---|
| | E | F | G | 2 | 3 |
| Water | To 100 | To 100 | To 100 | To 100 | To 100 |
| Retinyl Propionate | 0.32 | 0.32 | 0.32 | 0.32 | 0.32 |
| StarDesign^{™} Power ⁴ | 0.50 | 1.00 | 2.00 | 3.00 | 12.00 |
| Xanthan Gum | 0.20 | 0.20 | 0.20 | 0.20 | 0.20 |
| Isopropyl Myristate | 13.50 | 13.50 | 13.50 | 13.50 | 13.50 |
| Capric/Caprylic Triglyceride | 6.50 | 6.50 | 6.50 | 6.50 | 6.50 |
| Hexylresorcinol | 0.40 | 0.40 | 0.40 | 0.40 | 0.40 |
| Niacinamide | 3.00 | 3.00 | 3.00 | 3.00 | 3.00 |
| Glycerin | 2.00 | 2.00 | 2.00 | 2.00 | 2.00 |
| 1,3-Butylene glycol | 3.00 | 3.00 | 3.00 | 3.00 | 3.00 |
| Cetyl Alcohol | 0.50 | 0.50 | 0.50 | 0.50 | 0.50 |
| Disodium EDTA | 0.05 | 0.05 | 0.05 | 0.05 | 0.05 |
| Antioxidant | 0.20 | 0.20 | 0.20 | 0.20 | 0.20 |
| Preservative | 0.65 | 0.65 | 0.65 | 0.65 | 0.65 |

| | | | | | |
|---|---|---|---|---|---|
| *The level refers the level of the listed ingredient. 1: StarDesign^{™} Power, contains 10-40% of hydroxypropyl starch phosphate and 60-90% of sodium starch octenylsuccinate by weight, supplied by Cargill. | | | | | |

A series of skin care compositions were formulated according to Table 3 by following standard procedures.

The performances of the samples were evaluated by following same procedure as Example 1. The results were recorded in Table 4.

**Table 4.**

| Sample | Before storage at 55°C | After storage at 55°C |
|---|---|---|
| E | Stable emulsion | Phase separation |
| F | Stable emulsion | Phase separation |
| G | Stable emulsion | Phase separation |
| 2 | Stable emulsion | Stable emulsion |
| 3 | Stable emulsion | Stable emulsion |

It was surprisingly found to achieve a stable emulsion after storage at high temperature, the weight ratio of the total modified starches to the retinoid needs to be at least 7:1.

## Claims

1. A skin care composition comprising:
(a) retinoid;
(b) a first modified starch; and
(c) a second modified starch,
wherein the weight ratio of the total modified starches to the retinoid is at least 7:1, the retinoid comprises retinol, retinyl ester, or a mixture thereof, the first modified starch is nonionic starch, and the second modified starch is anionic starch.

2. The composition according to claim 1 wherein the retinoid is selected from retinyl palmitate, retinyl propionate, or a mixture thereof.

3. The composition according to claim 1 or 2 wherein the retinoid is present in amount of 0.005% to 3%, preferably in an amount of 0.1% to 0.5% by weight of the composition.

4. The composition according to any one of the preceding claims wherein the first modified starch is hydroxypropyl starch phosphate.

5. The composition according to any one of the preceding claims wherein the first modified starch is present in amount of 0.01 to 8%, preferably 0.2 to 2% by weight of the composition.

6. The composition according to any one of the preceding claims wherein the second modified starch comprises aluminium starch octenylsuccinate, sodium starch octenylsuccinate or a combination thereof.

7. The composition according to any one of the preceding claims wherein the second modified starch is present in amount of 0.01 to 12%, preferably 0.5 to 5% by weight of the composition.

8. The composition according to any one of the preceding claims wherein the weight ratio of the first modified starch to the second modified starch is 1:30 to 5:1, preferably 1:10 to 1:1.

9. The composition according to any one of the preceding claims wherein the weight ratio of the total modified starches to the retinoid is 7:1 to 200:1, preferably 8:1 to 50:1.

10. The composition according to any one of the preceding claims wherein the composition comprises a fatty ester, preferably a combination of fatty monoester and fatty polyester.

11. The composition according to claim 10 wherein the fatty monoester is monoester of C₁₀₋₂₀ fatty acid with C₁₋₆ monovalent alcohol or monoester of C₂₋₄ organic acid with C₈₋₁₈ monovalent alcohol, preferably the fatty monoester comprises myristyl propionate, cetyl propionate, isodecyl neopentanoate, isopropyl myristate, isopropyl palmitate, methyl myristate, methyl stearate, or a mixture thereof.

12. The composition according to claim 10 or 11 wherein the fatty polyester is ester of C₈₋₁₈ fatty acid with C₂₋₆ polyol or C₂₋₆ diol, preferably fatty polyester comprises capric/caprylic triglyceride.

13. The composition according to any one of claim 10 to 12 wherein the total amount of fatty ester is present in amount of 3 to 45%, preferably 12 to 28% by weight of the composition.

14. The composition according to claim 10 wherein the weight ratio of the total fatty esters to the total modified starches is no greater than 9:1, preferably 1:2 to 8:1.

15. The composition according to any one of the preceding claims wherein the composition is an oil-in-water emulsion.

## Patentansprüche

1. Hautpflegezusammensetzung, umfassend:
(a) Retinoid;
(b) eine erste modifizierte Stärke; und
(c) eine zweite modifizierte Stärke,
wobei das Gewichtsverhältnis der gesamten modifizierten Stärken zum Retinoid mindestens 7:1 beträgt, das Retinoid Retinol, Retinylester oder eine Mischung davon umfasst, die erste modifizierte Stärke eine nichtionische Stärke ist und die zweite modifizierte Stärke eine anionische Stärke ist.

2. Zusammensetzung nach Anspruch 1, wobei das Retinoid aus Retinylpalmitat, Retinylpropionat oder einer Mischung davon ausgewählt ist.

3. Zusammensetzung nach Anspruch 1 oder 2, wobei das Retinoid in einer Menge von 0,005 bis 3 Gew.-% der Zusammensetzung vorhanden ist, vorzugsweise in einer Menge von 0,1 bis 0,5 Gew.-%.

4. Zusammensetzung nach irgendeinem der vorhergehenden Ansprüche, wobei die erste modifizierte Stärke Hydroxypropylstärkephosphat ist.

5. Zusammensetzung nach irgendeinem der vorhergehenden Ansprüche, wobei die erste modifizierte Stärke in einer Menge von 0,01 bis 8 Gew.-% der Zusammensetzung vorhanden ist, vorzugsweise von 0,2 bis 2 Gew-%.

6. Zusammensetzung nach irgendeinem der vorhergehenden Ansprüche, wobei die zweite modifizierte Stärke Aluminiumstärkeoctenylsuccinat, Natriumstärkeoctenylsuccinat oder eine Kombination davon umfasst.

7. Zusammensetzung nach irgendeinem der vorhergehenden Ansprüche, wobei die zweite modifizierte Stärke in einer Menge von 0,01 bis 12 Gew.-% der Zusammensetzung vorhanden ist, vorzugsweise von 0,5 bis 5 Gew.-%.

8. Zusammensetzung nach irgendeinem der vorhergehenden Ansprüche, wobei das Gewichtsverhältnis der ersten modifizierten Stärke zur zweiten modifizierten Stärke 1:30 bis 5:1, vorzugsweise 1:10 bis 1:1, beträgt.

9. Zusammensetzung nach irgendeinem der vorhergehenden Ansprüche, wobei das Gewichtsverhältnis der gesamten modifizierten Stärken zu dem Retinoid 7:1 bis 200:1, vorzugsweise 8:1 bis 50:1, beträgt.

10. Zusammensetzung nach irgendeinem der vorhergehenden Ansprüche, wobei die Zusammensetzung einen Fettsäureester, vorzugsweise eine Kombination aus einem Fettsäuremonoester und einem Fettsäurepolyester, umfasst.

11. Zusammensetzung nach Anspruch 10, wobei der Fettsäuremonoester ein Monoester einer C₁₀₋₂₀-Fettsäure mit einwertigem C₁₋₆-Alkohol oder ein Monoester einer organischen C₂₋₄-Säure mit einwertigem C₈₋₁₈-Alkohol ist, wobei der Fettsäuremonoester vorzugsweise Myristylpropionat, Cetylpropionat, Isodecylneopentanoat, Isopropylmyristat, Isopropylpalmitat, Methylmyristat, Methylstearat oder eine Mischung davon umfasst.

12. Zusammensetzung nach Anspruch 10 oder 11, wobei der Fettsäurepolyester ein Ester einer C₈₋₁₈-Fettsäure mit C₂₋₆-Polyol oder C₂₋₆-Diol ist, vorzugsweise der Fettsäurepolyester Capric/Capryl-Triglycerid umfasst.

13. Zusammensetzung nach irgendeinem der Ansprüche 10 bis 12, wobei der gesamte Fettsäureester in einer Menge von 3 bis 45 Gew.-% der Zusammensetzung vorhanden ist, vorzugsweise von 12 bis 28 Gew.-%.

14. Zusammensetzung nach Anspruch 10, wobei das Gewichtsverhältnis der gesamten Fettsäureester zu den gesamten modifizierten Stärken nicht größer als 9:1, vorzugsweise 1:2 bis 8:1, ist.

15. Zusammensetzung nach irgendeinem der vorhergehenden Ansprüche, wobei die Zusammensetzung eine Öl-in-Wasser-Emulsion ist.

## Revendications

1. Composition de soins pour la peau comprenant:
(a) un rétinoïde;
(b) un premier amidon modifié; et
(c) un second amidon modifié,
dans laquelle le rapport pondéral des amidons modifiés totaux au rétinoïde est d'au moins 7:1, le rétinoïde comprend du rétinol, un ester de rétinyle ou un mélange de ceux-ci, le premier amidon modifié est un amidon non ionique et le second amidon modifié est un amidon anionique.

2. Composition selon la revendication 1, dans laquelle le rétinoïde est choisi parmi le palmitate de rétinyle, le propionate de rétinyle ou un mélange de ceux-ci.

3. Composition selon la revendication 1 ou 2, dans laquelle le rétinoïde est présent en une quantité de 0,005 % à 3 %, de préférence en une quantité de 0,1 % à 0,5 % en poids de la composition.

4. Composition selon l'une quelconque des revendications précédentes, dans laquelle le premier amidon modifié est du phosphate d'hydroxypropylamidon.

5. Composition selon l'une quelconque des revendications précédentes, dans laquelle le premier amidon modifié est présent en une quantité de 0,01 à 8 %, de préférence de 0,2 à 2 %, en poids de la composition.

6. Composition selon l'une quelconque des revendications précédentes, dans laquelle le second amidon modifié comprend de l'octénylsuccinate d'amidon d'aluminium, de l'octénylsuccinate d'amidon de sodium ou une combinaison de ceux-ci.

7. Composition selon l'une quelconque des revendications précédentes, dans laquelle le second amidon modifié est présent en une quantité de 0,01 à 12 %, de préférence de 0,5 à 5 %, en poids de la composition.

8. Composition selon l'une quelconque des revendications précédentes, dans laquelle le rapport pondéral du premier amidon modifié au second amidon modifié est de 1:30 à 5:1, de préférence de 1:10 à 1:1.

9. Composition selon l'une quelconque des revendications précédentes, dans laquelle le rapport pondéral des amidons modifiés totaux au rétinoïde est de 7:1 à 200:1, de préférence de 8:1 à 50:1.

10. Composition selon l'une quelconque des revendications précédentes, dans laquelle la composition comprend un ester gras, de préférence une combinaison de monoester gras et de polyester gras.

11. Composition selon la revendication 10, dans laquelle le monoester gras est un monoester d'acide gras en C₁₀₋₂₀ avec un alcool monovalent en C₁₋₆ ou un monoester d'acide organique en C₂₋₄ avec un alcool monovalent en C₈₋₁₈, de préférence le monoester gras comprend du propionate de myristyle, du propionate de cétyle, du néopentanoate d'isodécyle, du myristate d'isopropyle, du palmitate d'isopropyle, du myristate de méthyle, du stéarate de méthyle ou un mélange de ceux-ci.

12. Composition selon la revendication 10 ou 11, dans laquelle le polyester gras est un ester d'acide gras en C₈₋₁₈ avec un polyol en C₂₋₆ ou un diol en C₂₋₆, de préférence le polyester gras comprend un triglycéride caprique/caprylique.

13. Composition selon l'une quelconque des revendications 10 à 12, dans laquelle la quantité totale d'ester gras est présent en une quantité de 3 à 45 %, de préférence de 12 à 28 %, en poids de la composition.

14. Composition selon la revendication 10, dans laquelle le rapport pondéral des esters gras totaux aux amidons modifiés totaux n'est pas supérieur à 9:1, de préférence 1:2 à 8:1.

15. Composition selon l'une quelconque des revendications précédentes, dans laquelle la composition est une émulsion huile-dans-eau.
